(19) Europäisches Patentamt
European Patent Office
Office européen des brevets

(11) **EP 3 817 714 B1**

(12) **EUROPEAN PATENT SPECIFICATION**

(45) Date of publication and mention
of the grant of the patent:
**01.10.2025 Bulletin 2025/40**

(21) Application number: **19755780.4**

(22) Date of filing: **08.08.2019**

(51) International Patent Classification (IPC):
***A61K 8/49*** (2006.01)  ***A61Q 11/00*** (2006.01)

(52) Cooperative Patent Classification (CPC):
**A61K 8/4966; A61Q 11/00;** A61K 2800/87;
A61K 2800/88; A61K 2800/882

(86) International application number:
**PCT/US2019/045674**

(87) International publication number:
**WO 2020/033661 (13.02.2020 Gazette 2020/07)**

(54) **WHITENING COMPOSITIONS AND METHODS FOR THE SAME**

BLEICHUNGSZUSAMMENSETZUNGEN UND VERFAHREN ZUR HERSTELLUNG DAVON

COMPOSITIONS DE BLANCHIMENT ET PROCÉDÉS POUR CELLES-CI

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **10.08.2018 US 201862717019 P**

(43) Date of publication of application:
**12.05.2021 Bulletin 2021/19**

(73) Proprietor: **Colgate-Palmolive Company
New York, NY 10022 (US)**

(72) Inventors:
• **HUANG, Chun
Somerset, New Jersey 8873 (US)**

• **XU, Guofeng
Plainsboro, New Jersey 8536 (US)**

(74) Representative: **Sonnenhauser, Thomas Martin
Wuesthoff & Wuesthoff
Patentanwälte und Rechtsanwalt PartG mbB
Schweigerstraße 2
81541 München (DE)**

(56) References cited:
WO-A1-2017/048217    US-A- 5 310 563
US-A1- 2009 042 756    US-A1- 2010 012 891
US-A1- 2010 015 251    US-A1- 2018 168 973

## Description

### BACKGROUND

Conventional oral care products (e.g., toothpastes, whitening gels, whitening trays, etc.) and whitening compositions thereof are often utilized to whiten teeth. For example, conventional whitening gels including hydrogen peroxide are often utilized to oxidize chromophores on surfaces of teeth as well as chromophores that have diffused within the enamel to thereby whiten the teeth. While whitening gels including hydrogen peroxide have proven to be effective for whitening teeth, different chromophores on the surfaces are often oxidized at varying rates and/or via varying mechanisms. Additionally, hydrogen peroxide often exhibits relatively decreased stability as compared to other whitening agents, and the amount of hydrogen peroxide that may be utilized in oral care products may often be regulated. Accordingly, oral care products may often incorporate hydrogen peroxide and at least one additional whitening agent to address the deficiencies of utilizing hydrogen peroxide alone.

While the oral care products incorporating a variety of whitening agents have demonstrated increased efficacy in whitening teeth, there is a desire to discover and develop new whitening agents having improved whitening and increased reactivity to reduce periods of treatment, and to address regulations associated with the use of hydrogen peroxide.

What is needed, then, are improved oral care products and whitening compositions having increased stability and whitening efficacy. US 2010/0012891 A1 discloses a non-cytotoxic chlorine dioxide composition and a mixed agent bleaching composition for tooth whitening and methods of use of the compositions.

### BRIEF SUMMARY

The foregoing and/or other aspects and utilities embodied in the present disclosure may be achieved by providing a whitening composition as defined in the claims.

The chloroisocyanurate includes dichloroisocyanurate as defined in the claims and may further include a trichloroisocyanurate.

The dichloroisocyanurate is anhydrous sodium dichloroisocyanurate and/or sodium dichloroisocyanurate dihydrate as defined in the claims.

In at least one implementation, the base gel may include a polysiloxane fluid.

In at least one implementation, the base gel may include a pressure sensitive adhesive, optionally, the pressure sensitive adhesive may be a polyorganosiloxane.

In at least one implementation, the base gel may include a thickener, optionally, the thickener may include one or more of colloidal silica, fumed silica, and a cross-linked polyvinylpyrrolidone (PVP) polymer.

The base gel includes an adhesion enhancing agent as defined in the claims, optionally, the adhesion enhancing agent is white petrolatum as defined in the claims.

In at least one implementation, the aqueous component may include a hydrophilic polymer, optionally, the hydrophilic polymer may include a carboxypolymethylene.

In at least one implementation, the aqueous component may include one or more of a basifying agent and a buffering agent, optionally, the basifying agent may include sodium hydroxide, further optionally, the buffering agent may include sodium phosphate monobasic.

In at least one implementation, the whitening composition may be free or substantially free of peroxides and/or peroxide compounds.

In at least one implementation, a weight ratio of the anhydrous component to the aqueous component may be from 0.1:1 to 1.5:1.

The foregoing and/or other aspects and utilities embodied in the present disclosure may be achieved by providing an oral care product including any one of the whitening compositions as defined in the claims, and a dental tray. The dental tray of the oral care product as defined in the claims defines a reservoir configured to contain any one of the whitening compositions as defined in the claims.

In at least one implementation, the oral care product may include a first syringe and a second syringe. The first syringe may define a first chamber, and the anhydrous component of the whitening composition may be disposed in the first chamber. The second syringe may define a second chamber, and the aqueous component may be disposed in the second chamber.

In at least one implementation, the oral care product may include a dual-chamber syringe defining a first chamber and a second chamber. The anhydrous component and the aqueous component of the whitening composition may be disposed in the first chamber and the second chamber of the dual-chamber syringe, respectively.

The foregoing and/or other aspects and utilities embodied in the present disclosure may be achieved by providing a method for whitening teeth as defined in the claims. The method includes contacting the anhydrous component and the aqueous component of any one of the whitening compositions as defined in the claims with one another to form a mixture,

and contacting the mixture with surfaces of the teeth.

In at least one implementation, the method may further include generating a whitening agent after contacting the anhydrous component and the aqueous component with one another, optionally, the whitening agent is a hypochlorite.

In at least one implementation, contacting the mixture with the surfaces of the teeth may include disposing the mixture in a dental tray.

In at least one implementation, contacting the mixture with the surfaces of the teeth may further include disposing the dental tray the teeth to contact the whitening agent with the surfaces of the teeth.

In at least one implementation, the method may further include contacting the mixture with the surfaces of the teeth for at least 30 seconds, preferably at least 1 minute, more preferably at least 2 minutes.

## DETAILED DESCRIPTION

As used throughout, ranges are used as shorthand for describing each and every value that is within the range. Any value within the range may be selected as the terminus of the range. In the event of a conflict in a definition in the present disclosure and that of a cited reference, the present disclosure controls.

Unless otherwise specified, all percentages and amounts expressed herein and elsewhere in the specification should be understood to refer to percentages by weight. The amounts given are based on the active weight of the material.

Additionally, all numerical values are "about" or "approximately" the indicated value, and take into account experimental error and variations that would be expected by a person having ordinary skill in the art. It should be appreciated that all numerical values and ranges disclosed herein are approximate values and ranges, whether "about" is used in conjunction therewith. It should also be appreciated that the term "about," as used herein, in conjunction with a numeral refers to a value that may be $\pm$ 0.01% (inclusive), $\pm$ 0.1% (inclusive), $\pm$ 0.5% (inclusive), $\pm$ 1% (inclusive) of that numeral, $\pm$ 2% (inclusive) of that numeral, $\pm$ 3% (inclusive) of that numeral, $\pm$ 5% (inclusive) of that numeral, $\pm$ 10% (inclusive) of that numeral, or $\pm$ 15% (inclusive) of that numeral. It should further be appreciated that when a numerical range is disclosed herein, any numerical value falling within the range is also specifically disclosed.

The present inventors have surprisingly and unexpectedly discovered that a two-component whitening composition including an anhydrous component having a chloroisocyanurate and an aqueous component exhibits increased whitening efficacy as compared to hydrogen peroxide based whitening compositions.

## COMPOSITIONS

The whitening composition may be a multi-phase or multi-component whitening composition. For example, the whitening composition may be a multi-component whitening composition including at least two, three, or more components or phases maintained separate from one another until the point of use. As further described herein, the components of the whitening composition may be contacted with one another to generate a whitening agent (e.g., hypochlorite, hypochlorous acid, etc.). In an exemplary implementation, the whitening composition is a two-component whitening composition including an anhydrous component and an aqueous component, and the anhydrous component and the aqueous component may be mixed, combined, or otherwise contacted with one another to generate the whitening agent.

Illustrative oral care products of the present disclosure may be or include, but are not limited to, a toothpaste (dentifrice), a prophylactic paste, a tooth powder, a tooth polish, a tooth gel (e.g., a whitening gel), a chewing gum, a lozenge, a mouthwash, a whitening strip, a paint-on gel, varnish, veneer, and tube, syringe and/or dental tray including a gel or paste, or a gel or paste coated on an application support such as dental floss or a toothbrush (e.g., a manual, electric, sound, a combination thereof or ultrasound toothbrush). The oral care product of the invention is defined in the claims and comprises a whitening composition and a dental tray as defined in the claims. In an exemplary implementation, the oral care product disclosed herein includes a two-component whitening composition having an anhydrous component and an aqueous component, one or more syringes or a dual-chamber syringe configured to contain the whitening composition or the components (aqueous and anhydrous components) thereof, or any combination thereof.

The anhydrous component and the aqueous component of the whitening composition may be maintained separate from one another until the time of use; and at the time of use, the anhydrous component and the aqueous component may be combined, mixed, or otherwise contacted with one another. In at least one implementation, the anhydrous component and the aqueous component may be maintained in separate vessels or containers for storage, transportation, and/or sale before use. For example, the anhydrous component may be disposed or maintained in a first vessel or container and the aqueous component may be disposed or maintained in a second vessel or container, and the contents of the first and second vessels may be combined with one another shortly prior to or at the time of use. Illustrative vessels or containers may be or include, but are not limited to, dual-chambered or double-tubed syringes, two or more syringes, dual-chambered tubes (e.g., toothpaste tubes), two or more tubes, and the like.

The anhydrous component and the aqueous component may be contacted with one another in any amount or ratio sufficient to generate the whitening agent (e.g., hypochlorite, hypochlorous acid, etc.). In at least one implementation, a weight ratio of the anhydrous component to the aqueous component or the aqueous component to the anhydrous component may be from 0.1:1 to 1.5:1. For example, the weight ratio of the anhydrous component to the aqueous component or the aqueous component to the anhydrous component may be from greater than 0:1, 0.1:1, 0.2:1, 0.3:1, 0.4:1, 0.5:1, 0.6:1, 0.7:1, 0.8:1, or 0.9:1 to 1.1:1, 1.2:1, 1.3:1, 1.4:1, 1.5:1, 1.6:1, 1.7:1, 1.8:1, 1.9:1, or 2.0:1. In another example, the weight ratio of the anhydrous component to the aqueous component or the aqueous component to the anhydrous component may be from greater than 0:1 to 2.0:1, 0.1:1 to 1.9:1, 0.2:1 to 1.8:1, 0.3:1 to 1.7:1, 0.4:1 to 1.6:1, 0.5:1 to 1.5:1, 0.6:1 to 1.4:1, 0.7:1 to 1.3:1, 0.8:1 to 1.2:1, 0.9:1 to 1.1:1, or 0.95:1 to 1.05:1.

In at least one implementation, the oral care products or the whitening compositions thereof disclosed herein may be free or substantially free of peroxides or peroxide compounds. As used herein, the terms "free" or "substantially free" may refer to a composition that contains less than 0.1 wt%, less than 0.05 wt%, less than 0.01 wt%, less than 0.005 wt%, or less than 0.0001 wt%. Accordingly, the tooth whitening effect of the oral care products or the whitening compositions thereof may be provided by the non-peroxide whitening agents rather than by the presence of any peroxide whitening agents.

*Anhydrous Component*

The anhydrous component includes chloroisocyanurates and a base gel as further defined in the claims. The anhydrous component or phase may be free or substantially free of water. As used herein, "free" or "substantially free" may refer to a composition, component, or phase that contains water in an amount of less than 10.0 wt%, less than 5.0 wt%, less than 3.0 wt%, less than 1.0 wt%, less than 0.1 wt%, less than 0.05 wt%, less than 0.01 wt%, less than 0.005 wt%, or less than 0.0001 wt%, based on a total weight of the whitening composition, component, or phase.

The chloroisocyanurate includes dichloroisocyanurate, which is anhydrous sodium dichloroisocyanurate and/or sodium dichloroisocyanurate dihydrate as defined in the claims. The chloroisocyanurates may also further include, but are not limited to, one or more alkali metal salts of dichloroisocyanurates, trichloroisocyanurates, and the like, or combinations thereof. Illustrative chloroisocyanurates may be or include, but are not limited to, anhydrous sodium dichloroisocyanurate (NaDCC), sodium dichloroisocyanurate dihydrate (NaDCC·$H_2$O), potassium dichloroisocyanurate (KDCC), trichloroisocyanuric acid (TCCA), and the like, or combinations thereof. In an exemplary implementation, the chloroisocyanurate includes NaDCC·$H_2$O or dichloroisocyanuric acid sodium salt dihydrate.

The chloroisocyanurate may be present in the two-component whitening composition or the anhydrous component thereof in an effective amount to whiten teeth. For example, the amount of the chloroisocyanurate present in the two-component whitening composition or the anhydrous component thereof may be an amount effective to provide relatively greater teeth whitening than a control whitening composition that does not include the chloroisocyanurate, such as a hydrogen peroxide (HP) based whitening composition (e.g., 6% HP whitening gel composition).

The amount of the chloroisocyanurate present in the two-component whitening composition or the anhydrous component thereof is from 0.01 weight % to 3.0 weight % as defined in the claims. For example, the amount of the chloroisocyanurate present in the two-component whitening composition or the anhydrous component thereof may be from 0.01 weight %, 0.05 weight %, 0.10 weight %, 0.15 weight %, 0.20 weight %, 0.25 weight %, 0.30 weight %, 0.35 weight %, 0.40 weight %, 0.45 weight %, 0.50 weight %, 0.55 weight %, 0.60 weight %, or 0.70 weight % to 0.80 weight %, 0.90 weight %, 1.0 weight %, 1.10 weight %, 1.20 weight %, 1.30 weight %, 1.40 weight %, 1.50 weight %, 1.60 weight %, 1.70 weight %, 1.80 weight %, 1.90 weight %, 2.0 weight %, 2.1 weight %, 2.2 weight %, 2.3 weight %, 2.4 weight %, 2.5 weight %, 2.6 weight %, 2.7 weight %, 2.8 weight %, 2.9 weight %, or 3.0 weight %, based on a total weight of the whitening composition or the anhydrous component thereof. In some implementations, the amount of the chloroisocyanurate present in the whitening composition or the anhydrous component thereof is such that a unit dose of the solid composition does not exceed 2.2 mg of the chloroisocyanurate per day per kg weight of the user.

As discussed above, the anhydrous component may include one or more chloroisocyanurates and/or a base gel. In an exemplary implementation, the anhydrous component includes one or more chloroisocyanurates and the base gel. The base gel may be present in an amount of greater than or equal to 60 weight %, greater than or equal to 65 weight %, greater than or equal to 70 weight %, greater than or equal to 75 weight %, greater than or equal to 80 weight %, greater than or equal to 85 weight %, greater than or equal to 90 weight %, greater than or equal to 95 weight %, greater than or equal to 96 weight %, greater than or equal to 97 weight %, greater than or equal to 98 weight %, greater than or equal to 99 weight %, or greater than or equal to 99.5 weight %, based on a total weight of the anhydrous component.

The base gel includes one or more adhesion enhancing agents configured to increase adhesion of the whitening composition to surfaces of the oral cavity as defined in the claims. For example, the adhesion enhancing agent may be configured to increase adhesion of the whitening composition to surfaces of teeth (e.g., enamel). Illustrative adhesion enhancing agents may be or include, but are not limited to, inorganic, organic, natural, and/or synthetic materials and/or polymers, and the like, or combinations thereof.

The inorganic materials and/or polymers may be or include, but are not limited to, amorphous silica compounds, such as colloidal silica compounds. Illustrative amorphous silica compounds may include but are not limited to, CAB-O-SIL® Fumed Silica, commercially available from Cabot Corporation of Boston, MA, SYLODENT® 15, commercially available from Grace Corporation of Colombia, MD, and the like, and combinations thereof. In at least one implementation, the inorganic materials and/or polymers may be treated such that the surface thereof is compatible with one or more hydrophobic components of the whitening composition.

The organic materials and/or polymers may be or include, but are not limited to, waxes (e.g., bees' wax), mineral oil, gelled mineral oils, petrolatum, white petrolatum, shellac, versagel (blend of liquid paraffin, butenefethylenelstyrene hydrogenated copolymer) polyethylene waxes, microcrystalline waxes, polyisobutene, polyvinyl pyrrolidone/vinyl acetate copolymers, insoluble polyacrylate copolymers, and the like, and combinations thereof. Illustrative gelled mineral oils may include, but are not limited to, a blend or combination of mineral oil and polyethylene (e.g., plastigel). The adhesion enhancing agent is white petrolatum as defined in the claims. In yet another implementation, the adhesion enhancing agent may include PLASTIGEL® 5, which is a blend of 5% polyethylene in mineral oil, and is commercially available from Pharmaceutical Resources/Lyne Laboratories, Inc. of Brockton, MA. In another implementation, the adhesion enhancing agent may include PLASTIGEL® 5 and mineral oil. Other suitable gelled mineral oils or plastigels can be prepared in accordance with the teachings of Thau et al., "A New Procedure for the Preparation of Polyethylene-Mineral Oil Gels," J. Soc. Cosmetic Chemists, 16, 359-363 (1965).

The adhesion enhancing agents may include, but are not limited to, liquid hydrophilic polymers including polyethylene glycols, nonionic polymers of ethylene oxide, represented by the formula (1),

$$HOCH_2(CH_2OCH_2)_nCH_2OH \qquad (1),$$

where n represents the average number of oxyethylene groups. Polyethylene glycols are commercially available from Dow Chemical Corporation, and are designated by a number such as 200, 300, 400, 600, 2000, which represents the approximate average molecular weight of the polymer.

The adhesion enhancing agents may also include, but are not limited to, nonionic block copolymers of ethylene oxide and propylene oxide represented by the formula (2),

$$(\text{ethylene oxide})_x\text{-}(\text{propylene oxide})_y \qquad (2)$$

where x is an integer from 80 to 150 (e.g., x = 100-130, or 118), and y is an integer from 30 to 80 (e.g., y= 60-70, or 66). The block co-polymer of ethylene oxide and propylene oxide may have an average molecular weight greater than or equal to 2,000 Da and less than or equal to 20,000 Da. For example, the molecular weight of the block co-polymer of ethylene oxide and propylene oxide may be from 8,000 Da to 13,000 Da. In another example, the molecular weight of the block co-polymer of ethylene oxide and propylene oxide may be from 9,800 Da or 10,000 Da. In yet another example, the molecular weight of the block co-polymer of ethylene oxide and propylene oxide may be from 8,000 Da to 10,000 Da. In at least one implementation, the base gel does not include a block co-polymer of ethylene oxide and propylene oxide having a molecular weight less than 5,000 Da. For example, at least 99.5%, at least 99.0%, or at least 99.9% of the block co-polymer of ethylene oxide and propylene oxide present in the base gel has a molecular weight greater than or equal to 5,000 Da. The block copolymer may be selected such that the ethylene oxide constituent includes from 65 to 75% by weight of the copolymer molecule.

The adhesion enhancing agents of the base gel may include hydrophobic polymers, such as siloxane polymers, which are also generally known in the art as "silicone" polymers. Illustrative silicone-based hydrophobic polymers may be or include, but are not limited to, polyorganosiloxane, polydiorganosiloxane, and the like, and combinations thereof. In at least one implementation, the adhesion enhancing agent includes at least one silicon pressure sensitive adhesive (PSA). Such PSAs may be pressure sensitive hydrophobic polymers specifically designed for pharmaceutical use and are permeable to many drug compounds and find application for the transdermal application of various compounds. In some implementations, the silicone polymers are the copolymer product of mixing a silanol terminated polydiorganosiloxane such as polydimethyl siloxane with a silanol-containing silicone resin whereby the silanol groups of the polydiorganosiloxane undergo a condensation reaction with the silanol groups of the silicone resin so that the polydiorganosiloxane is lightly crosslinked by the silicone resin (that is, the polydiorganosiloxane chains are bonded together through the resin molecules to give chain branching and entanglement and/or a small amount of network character) to form the silicone hydrophobic polymers. In at least one implementation, the adhesion enhancing agents or the hydrophobic polymers thereof are available from the Dow-Corning Company under the brand name BIO-PSA. The modification of a ratio of silicone resin to polydiorganosiloxane modifies the tackiness of the polymer. This ratio may be in the range of 70:30 to 50:50. For example, the BIO-PSA silicone commercially available from Dow-Corning is available in varying silicone resin to silicone polymer ratios, namely, 65/35 (low tack), 60/40 (medium tack), 55/45 (high tack). Such a polyorganosiloxane PSA is available dissolved in either ethyl acetate solvent or dimethicone. In at least one implementation, the adhesion enhancing agent may include Silicone Adhesive 8-7016, commercially available from Dow Corning Corporation of Midland, MI.

The adhesion enhancing agents of the base gel may include siloxane polymers in the form of a fluid, such as polysiloxane fluids. Illustrative polysiloxane fluids may be or include, but are not limited to, those having a viscosity at 25°C of 1 to 1,000 mPa-s, 2 to 500 mPa-s, or 20 to 400 mPa-s. The polysiloxane fluids may be linear or cyclic, and may be substituted with a variety of substituents, such as methyl, ethyl and phenyl substituents. In at least one implementation, the polysiloxane fluid may be Q7-9120, commercially available from Dow Corning Corporation of Midland, MI.

The one or more adhesion enhancing agents may be present in an amount of from 60 weight % to 90 weight %, based on a total weight of the two-component whitening composition or the base gel thereof. For example, the amount of the one or more adhesion enhancing agents present may be from 60 weight %, 65 weight %, 70 weight %, or 72.5 weight % to 77.5 weight %, 80 weight %, 85 weight %, or 90 weight %. In another example, the amount of the one or more adhesion enhancing agents present may be from 60 weight % to 90 weight %, 65 weight % to 85 weight %, 70 weight % to 80 weight %, or 72.5 weight % to 77.5 weight %, based on a total weight of the two-component whitening composition or the base gel thereof.

The base gel may also include a thickening system or one or more thickeners or viscosity control agents. The one or more thickeners may be any orally acceptable thickener or thickening agent configured to control the viscosity of the two-component whitening composition or the anhydrous component thereof. Illustrative thickeners may be or include, but are not limited to, colloidal silica, fumed silica, a cross-linked polyvinylpyrrolidone (PVP) polymer, and the like, and mixtures or combinations thereof. In at least one implementation, the thickening system includes a cross-linked polyvinylpyrrolidone (PVP) polymer. The thickening system may also include POLYPLASDONE® XL 10F, which is commercially available from Ashland Inc. of Covington, KY. Illustrative thickeners may also be or include, but are not limited to, carbomers (e.g., carboxyvinyl polymers), carrageenans (e.g., Irish moss, carrageenan, iota-carrageenan, etc.), high molecular weight polyethylene glycols (e.g., CARBOWAX®, which is commercially available from The Dow Chemical Company of Midland, MI), cellulosic polymers, hydroxyethylcellulose, carboxymethylcellulose, and salts thereof (e.g., CMC sodium), natural gums (e.g., karaya, xanthan, gum arabic, and tragacanth), colloidal magnesium aluminum silicate, and the like, and mixtures or combinations thereof.

In a more typical implementation, the thickening system may include an organic polymer, which may also be configured as an adhesion enhancing agent. Illustrative organic polymers may be or include, but are not limited to, hydrophilic polymers, such as carbomers, such as carboxymethylene polymers, such as acrylic acid polymers, and acrylic acid copolymers. Carboxypolymethylene is a slightly acidic vinyl polymer with active carboxyl groups. In a typical implementation, the thickening system includes a carboxypolymethylene, such as CARBOPOL® 974 and/or 980, which are commercially available from Noveon, Inc. of Cleveland, OH.

In at least one implementation, the thickening system may include a single thickener. For example, the thickening system may include the cross-linked polyvinylpyrrolidone (PVP) polymer or an organic polymer (e.g., CARBOPOL®). In another implementation, the thickening system may include a plurality of thickeners. For example, the thickening system may include the cross-linked PVP polymer and the organic polymer.

The thickening system or the one or more thickeners thereof may be present in an amount of from 15 weight % to 35 weight %, based on a total weight of the two-component whitening composition or the base gel thereof. For example, the amount of the one or more thickeners present may be from 15 weight %, 20 weight %, or 22.5 weight % to 27.5 weight %, 30 weight %, or 35 weight %, based on a total weight of the two-component whitening composition or the base gel thereof.

*Aqueous Component*

The aqueous component of the two-component whitening composition includes water as defined in the claims. Water of the whitening composition and the aqueous component thereof may be deionized water, demineralized water, and/or softened water. Water of the aqueous component may be separate from the water of other components of the whitening composition. Water may make up the balance of the aqueous component of the two-component whitening composition. For example, the amount of water in the solid cleansing composition may be greater than or equal to 60 weight %, greater than or equal to 65 weight %, greater than or equal to 70 weight %, greater than or equal to 75 weight %, greater than or equal to 80 weight %, greater than or equal to 85 weight %, greater than or equal to 90 weight %, greater than or equal to 92 weight %, greater than or equal to 94 weight %, greater than or equal to 96 weight %, greater than or equal to 97 weight %, greater than or equal to 98 weight %, greater than or equal to 99 weight %, or greater than or equal to 99.5 weight %, based on a total weight of the aqueous component. The amount of water in the whitening composition or the aqueous component thereof may include free water added and/or water introduced with other components or materials of the whitening composition or the aqueous component thereof. For example, the amount of water in the whitening composition or the aqueous component thereof may include free water and water associated with a pH modifier, such as a base (e.g., 50% sodium hydroxide solution).

The aqueous component may also include any one or more of the thickeners or viscosity control agents discussed above with respect to the based gel. For example, in at least one implementation, the aqueous component includes an organic polymer, such as a carboxypolymethylene, CARBOPOL® 974, and/or CARBOPOL® 980.

The aqueous component may include one or more pH modifying agents. For example, the aqueous component may include one or more acidifying agents and/or one or more basifying agents configured to reduce and/or increase the pH thereof, respectively. Illustrative acidifying agents and/or one or more basifying agents may be or include, but are not limited to, an alkali metal hydroxide, such as sodium hydroxide and/or potassium hydroxide, citric acid, hydrochloric acid, or the like, or combinations thereof.

The aqueous component may also include one or more buffering agents configured to control or modulate the pH within a predetermined or desired range. Illustrative buffering agents may include, but are not limited to, sodium bicarbonate, sodium phosphate, sodium carbonate, sodium acid pyrophosphate, sodium citrate, and mixtures thereof. Sodium phosphate may include, monosodium phosphate ($NaH_2PO_4$), disodium phosphate ($Na_2HPO_4$), trisodium phosphate ($Na_3PO_4$), and mixtures thereof. In a typical implementation, the buffering agent may be anhydrous sodium phosphate dibasic or disodium phosphate and/or sodium phosphate monobasic. In another implementation, the buffering agent includes anhydrous sodium phosphate dibasic or disodium phosphate, and phosphoric acid (e.g., syrupy phosphoric acid; 85%-Food Grade).

In at least one implementation, the acidifying, basifying, and/or buffering agents may be included in the aqueous component to provide a generally neutral pH or an orally acceptable pH range. In another implementation, the acidifying, buffering, and/or buffering agents may be included in the aqueous component with a pH of from 2 to 7, 3 to 7, 4 to 7, 6 to 7, or 7 to 9. Any orally acceptable pH modifying agent may be used, including without limitation carboxylic, phosphoric, and sulfonic acids, acid salts (e.g., monosodium citrate, disodium citrate, monosodium malate, etc.), alkali metal hydroxides, such as sodium hydroxide, carbonates, such as sodium carbonate, bicarbonates, sesquicarbonates, borates, silicates, phosphates (e.g., monosodium phosphate, trisodium phosphate, pyrophosphate salts, etc.), imidazole and mixtures thereof. The one or more pH modifying agents may be optionally present in an amount effective to maintain the whitening composition or a component thereof in an orally acceptable pH range.

### *Additional Ingredients*

It should be appreciated by one having ordinary skill in the art, that the whitening composition and/or the components (e.g., the aqueous or anhydrous components) thereof may include other additional ingredients/components. For example, the whitening composition and/or the components thereof may include one or more anticaries agents, one or more desensitizing agents, one or more viscosity modifiers, one or more diluents, one or more surface active agents (e.g., emulsifiers, foam modulators, etc.), one or more humectants, one or more mouth feel agents, one or more sweetening agents, one or more flavor agents, one or more colorants, one or more preservatives, and the like, or combinations and mixtures thereof. It should further be appreciated by one having ordinary skill in the art that while general attributes of each of the above categories of materials may differ, there may be some common attributes and any given material may serve multiple purposes within two or more of such categories of materials.

### *Whitening Agent*

As discussed above, the anhydrous component and the aqueous component may be mixed, combined, or otherwise contacted with one another to generate the whitening agent. In an exemplary implementation, the whitening agent is a hypochlorite, hypochlorous acid, or the like. In a typical implementation, the generation of the whitening agent from the whitening composition may be initiated by mixing, combining, or otherwise contacting the chloroisocyanurate of the anhydrous component with the aqueous component or water thereof. For example, contacting the chloroisocyanurate (e.g., sodium dichloroisocyanurate) with the aqueous component or water thereof may result in the hydrolysis of the chloroisocyanurate and the generation of hypochlorite (e.g., hypochlorous acid, sodium hypochlorite, etc.).

The amount or concentration of the hypochlorite generated by contacting the chloroisocyanurate of the anhydrous component with the aqueous component or water thereof may vary widely. The amount of the hypochlorite generated may be less than or equal to 2.2 mg/day/Kg weight of the user, less than or equal to 2.1 mg/day/Kg weight of the user, less than or equal to 2.0 mg/day/Kg weight of the user, less than or equal to 1.9 mg/day/Kg weight of the user, less than or equal to 1.8 mg/day/Kg weight of the user, less than or equal to 1.7 mg/day/Kg weight of the user, less than or equal to 1.6 mg/day/Kg weight of the user, less than or equal to 1.5 mg/day/Kg weight of the user, less than or equal to 1.4 mg/day/Kg weight of the user, less than or equal to 1.3 mg/day/Kg weight of the user, less than or equal to 1.2 mg/day/Kg weight of the user, less than or equal to 1 mg/day/Kg weight of the user, or less than or equal to 0.5 mg/day/Kg weight of the user.

The whitening agent may be generated within at least 3 minutes (min) from contacting the anhydrous component with the aqueous component or the water thereof. For example, the whitening agent of the two-component whitening composition may be generated in less than or equal to 3 min, less than or equal to 2.8 min, less than or equal to 2.6 min, less than or equal to 2.4 min, less than or equal to 2.2 min, less than or equal to 2.0 min, less than or equal to 1.8 min, less than or equal to 1.6 min, less than or equal to 1.4 min, less than or equal to 1.2 min, less than or equal to 1.0 min, less than or equal to 0.8 min, less than or equal to 0.6 min, or less than or equal to 0.4 min.

## METHODS

A method of storing the two-component whitening composition may include storing or maintaining the anhydrous component of the whitening composition in a first vessel or container, and storing or maintaining the aqueous component of the whitening composition in a second vessel or container. The first vessel or container may be a first syringe and the second vessel may be a second syringe separate from the first syringe. The first vessel may be a first chamber of a dual-chamber syringe, and the second vessel may be a second chamber of the dual-chamber syringe. It should be appreciated that maintaining the aqueous component and the anhydrous component separate from one another will prevent reactions therebetween, thereby extending the stability and shelf-life of the whitening composition.

The present disclosure also provides methods for whitening teeth in a human or animal subject with the oral care product and/or the whitening composition thereof as defined in the claims. As used herein "animal subject" may include higher order non-human mammals such as canines, felines, and horses. The method includes combining or contacting the aqueous component and the anhydrous component with one another as defined in the claims to generate the whitening agent. The method may also include generating the whitening agent via hydrolysis. The method may also include generating the whitening agent in a period of less than 3 min, less than 2 min, less than 1.5 min, less than 1 min, less than 0.5 min, or less. The method may also include contacting the surface of the teeth with the whitening composition and/or the whitening agent generated therefrom. In at least one implementation, contacting the surface of the teeth with the whitening composition may include brushing, flossing, irrigating, wiping, rinsing (lavage of the oral cavity), foam/gel and in-tray application, masticating, spraying, painting, and the like, or combinations thereof. In an exemplary implementation, contacting the surface of the teeth with the whitening composition may include disposing the whitening composition in a dental tray (e.g., a reservoir of the dental tray) and disposing the dental tray the teeth, or in other words, positioning the teeth in the dental tray that contains the whitening composition. The dental tray may be applied to the teeth and left for at least 30 seconds, at least 1 minute, at least 2 minutes, at least 5 minutes, typically at least 10 minutes, or more typically at least 30 minutes. After each treatment with the whitening composition, the teeth may be treated with a tooth desensitizing formulation. Illustrative desensitizing formulations may contain potassium nitrate, citric acid, citric acid salts, strontium chloride and the like.

The oral care product and/or the whitening composition thereof may be applied and/or contacted with the surfaces of the oral cavity at predetermined intervals. For example, on a daily basis, at least once a day for multiple days, or alternatively every other day. In another example, the oral care product and/or the whitening composition thereof may be applied and/or contacted with the surfaces of the teeth at least once a day, at least once every two days, at least once every three days, at least once every five days, at least once a week, at least once every two weeks, or at least once a month. The oral care product and/or the whitening composition thereof may be utilized for up to 2 weeks, up to 3 weeks, up to 4 weeks, up to 6 weeks, up to 8 weeks, or greater.

The dental tray disclosed herein may be of any conventional form, and may be formed from conventionally used polymers, such as thermoplastic polymers. Thermoset polymers also may be used. Accordingly, the dental tray may range from highly flexible to a low flexibility. The thermoplastic polymers are typically used. Illustrative thermoplastic polymers may be or include, but are not limited to, polyethylene and polypropylene polymers, their derivatives and copolymers, silicone elastomers, polyurethanes and derivatives, polycaprolactams, polystyrene and derivatives, polybutadiene and derivatives, polyisoprene and derivatives, and polymethacrylate and its derivatives, and the like, or combinations thereof.

All ingredients for use in the compositions described herein should be orally acceptable. As used herein, "orally acceptable" may refer any ingredient that is present in a composition as described in an amount and form which does not render the composition unsafe for use in the oral cavity.

## EXAMPLES

The examples and other implementations described herein are exemplary and not intended to be limiting in describing the full scope of compositions and methods of this disclosure. Equivalent changes, modifications and variations of specific implementations, materials, compositions and methods may be made within the scope of the present disclosure, with substantially similar results.

Example 1

Two-component whitening compositions were prepared, including an anhydrous component or phase and an aqueous component or phase. The anhydrous component of the whitening composition included an anhydrous chloroisocyanurate gel and the aqueous component of the whitening composition included an aqueous gel. The anhydrous component or anhydrous chloroisocyanurate gel was prepared by combining a chloroisocyanurate with a base silicon-fluid gel. Particularly, a 0.2 wt% sodium dichloroisocyanurate anhydrous gel (1) and a 2 wt% NaDCC anhydrous gel (2) were prepared by combining a base silicon-fluid gel with varying amounts of sodium dichloroisocyanurate dihydrate

(NaDCC·2H$_2$O). The base silicon-fluid gel was prepared by combining the ingredients/components according to Table 1. The base silicon-fluid gel was then combined with NaDCC·2H$_2$O according to Table 2 to prepare the test anhydrous chloroisocyanurate gels (1) and (2).

**Table 1**

| Base Silicon-Fluid Gel Composition | |
|---|---|
| **INGREDIENT/COMPONENT** | **Weight (%)** |
| Silicon Pressure Sensitive Adhesive (PSA) | 30.0 |
| Silicone Oil - about 350 cSt | 18.1 |
| Silicon Dioxide | 1.0 |
| Polyvinyl Pyrrolidone (PVP) | 25.0 |
| White Petrolatum - Mineral Oil | 25.0 |
| Flavor/Sweetener | 0.9 |
| **Total** | **100.0** |

**Table 2**

| Test Anhydrous Chloroisocyanurate Gel Compositions (1) and (2) | | |
|---|---|---|
| **INGREDIENT/COMPONENT** | **(1)** | **(2)** |
| Base Silicon-Fluid Gel | 98.0 wt% | 99.8 wt% |
| NaDCC·2H$_2$O | 2.0 wt% | 0.2 wt% |
| **Total** | **100.0 wt%** | **100.0 wt%** |

As discussed above, the aqueous component of the two-component whitening composition included an aqueous gel. The aqueous gel was prepared by combining the ingredients/components according to Table 3.

**Table 3**

| Aqueous Gel Composition | |
|---|---|
| **INGREDIENT/COMPONENT** | **Weight (%)** |
| CARBOPOL® 974P NF Polymer | 3.0 |
| Sodium Phosphate Monobasic, Monohydrate | 2.0 |
| Disodium Hydrogen Phosphate | 1.0 |
| Sodium Hydroxide (50% Soln) | 3.0 |
| Deionized Water | 91.0 |
| **Total** | **100.0** |

Example 2

The whitening efficacy of the two-component whitening compositions prepared in Example 1 and a control were evaluated in an *in vitro* study on artificially stained bovine central incisors individually mounted to resin blocks. The artificially stained bovine central incisors were obtained from Dental Product Testing Therametric Technologies, Inc. of Noblesville, IN. The control was a 6% hydrogen peroxide (HP) whitening gel composition prepared by combining the ingredients/components according to Table 4. The baseline L*, a*, b* values of the mounted bovine incisors were recorded with a spectrophotometer. The whiteness index, WIO, was calculated for each of the bovine incisors. The bovine incisors selected had an L* value between 57 and 64.

**Table 4**

| Control Whitening Gel Composition - 6% HP | |
|---|---|
| **Materials description** | **Weight (%)** |
| CARBOPOL® 974P NF Polymer | 3.0 |
| Sodium Phosphate Monobasic, Monohydrate | 1.0 |
| Disodium Hydrogen Phosphate | 2.0 |
| Sodium Hydroxide (50% Soln) | 3.0 |
| Hydrogen Peroxide (35%) | 17.1 |
| Water (di) | 73.9 |
| **Total** | **100.0** |

To evaluate the whitening efficacy of the two-component whitening compositions prepared in Example 1, the anhydrous component and the aqueous component of the two-component whitening compositions were loaded into a first tube and a second tube of a dual-vessel or double-tube syringe, respectively. Specifically, the first tube of the double-tube syringe was filled with the 0.2 wt% sodium dichloroisocyanurate anhydrous gel or the 2.0 wt% sodium dichloroisocyanurate anhydrous gel, and the second tube of the double-tube syringe was filled with the aqueous gel. The anhydrous and aqueous components were then discharged from the double-tube syringe and mixed with one another to form the whitening composition.

The control (6% HP whitening gel composition) or the mixed two-component whitening composition was then individually used to treat four artificially stained bovine incisors. Each treatment included contacting the four artificially stained bovine incisors with 300 mg of the control (6% HP whitening gel composition) or 300 mg of the mixed whitening composition, covering the teeth with aluminum foil, and soaking the bovine incisors in about 20 mL of artificial saliva for about 20 minutes.

The L*, a*, b* values after each treatment were compared to the baseline values measured before any treatment to calculate the whitening efficacy. The change in whiteness index ($\Delta W^*$) or whitening efficacy is summarized in Table 5. It should be appreciated that the whiteness index ($W^*$) is a measure of overall color change relative to pure white, and is given by formula (3), and the change in whiteness index ($\Delta W^*$) is measured by formula (4).

$$W^* = ((L^*-100)^2 + (a^*)^2 + (b^*)^2)^{1/2} \qquad (3)$$

$$\Delta W^* = W^*_{treated} - W^*_{baseline} \qquad (4)$$

**Table 4**

| Whitening Efficacy ($\Delta W$) After In Vitro Treatments | | | | | | | | |
|---|---|---|---|---|---|---|---|---|
| | **Number of Treatments** | | | | | | | |
| | **0** | **1** | **2** | **3** | **4** | **5** | **6** | **7** |
| **(1) 0.2 wt% NaDCC Anhydrous Gel + Aqueous Gel, ($\Delta W$)** | 0 | 1.084 ± 0.2097 | 1.943 ± 0.3229 | 2.829 ± 0.4117 | 2.851 ± 0.4878 | 3.190 ± 0.6665 | 3.352 ± 0.6004 | 3.538 ± 0.5718 |
| **(2) 2.0 wt% NaDCC Anhydrous Gel + Aqueous Gel, ($\Delta W$)** | 0 | 5.234 ± 1.1652 | 7.104 ± 0.9742 | 8.010 ± 1.1932 | 8.750 ± 1.2497 | 9.397 ± 1.1827 | 9.535 ± 1.1599 | 9.576 ± 1.0762 |
| **Control % HP Whitening Gel Composition, ($\Delta W$)** | 0 | 1.524 ± 0.0386 | 2.931 ± 0.5158 | 3.809 ± 0.4909 | 5.966 ± 0.0876 | 6.565 ± 1.0005 | 7.585 ± 1.4985 | 8.157 ± 1.2931 |

It should be appreciated that the greater the $\Delta W$ value, the greater the whitening efficacy or the whiter the teeth. As illustrated in Table 4, it was surprisingly and unexpectedly discovered that the two-component whitening composition including the 2 weight% NaDCC gel and the aqueous gel (2) exhibited relatively greater whitening efficacy ($\Delta W$) as

compared to the control, the 6% HP whitening gel composition. Table 4 further illustrated that the two-component whitening composition including the 2% NaDCC gel and the aqueous gel (2) was able to whiten teeth relatively faster than the control. Particularly, the bovine incisors treated with the two-component whitening composition including the 2% NaDCC gel and the aqueous gel (2) exhibited a ΔW of about 5.23 after a single treatment, which was five times greater than the ΔW (1.084) of the bovine incisor treated with the control after a single treatment.

**Claims**

1. A whitening composition, comprising:

   an anhydrous component comprising
   a base gel, wherein the base gel comprises an adhesion enhancing agent, which is white petrolatum, and a chloroisocyanurate, wherein the chloroisocyanurate includes dichloroisocyanurate, which is anhydrous sodium dichloroisocyanurate and/or sodium dichloroisocyanurate dihydrate, wherein the chloroisocyanurate is present in the anhydrous component in an amount of from 0.01 weight % to 3.0 weight %, based on a total weight of the anhydrous component; and
   an aqueous component comprising water.

2. The whitening composition of claim 1, wherein the dichloroisocyanurate is sodium dichloroisocyanurate dihydrate.

3. The whitening composition of any one of the preceding claims, wherein the base gel comprises a polysiloxane fluid.

4. The whitening composition of any one of the preceding claims, wherein the base gel comprises a pressure sensitive adhesive, optionally, the pressure sensitive adhesive is a polyorganosiloxane.

5. The whitening composition of any one of the preceding claims, wherein the base gel comprises a thickener, optionally, the thickener comprises one or more of colloidal silica, fumed silica, a cross-linked polyvinylpyrrolidone (PVP) polymer, or combinations thereof.

6. The whitening composition of any one of the preceding claims, wherein the aqueous component comprises a hydrophilic polymer, optionally, the hydrophilic polymer comprises a carboxypolymethylene.

7. The whitening composition of any one of the preceding claims, wherein the aqueous component comprises one or more of a basifying agent, a buffering agent, or combinations thereof, optionally, the basifying agent comprises sodium hydroxide, further optionally, the buffering agent comprises sodium phosphate monobasic.

8. The whitening composition of any one of the preceding claims, wherein the whitening composition is substantially free of peroxides and peroxide compounds.

9. The whitening composition of any one of the preceding claims, wherein a weight ratio of the anhydrous component to the aqueous component is from 0.1:1 to 1.5:1.

10. An oral care product, comprising:

    any one of the whitening compositions of claims 1 to 9; and
    a dental tray defining a reservoir configured to contain any one of the whitening compositions of claims 1 to 9.

11. A method for whitening teeth, comprising:

    contacting the anhydrous component and the aqueous component of any one of the whitening compositions of claims 1 to 9 with one another to form a mixture; and
    contacting the mixture with surfaces of the teeth.

12. The method of claim 11, wherein contacting the mixture with the surfaces of the teeth comprises disposing the mixture in a dental tray.

13. The method of claim 12, wherein contacting the mixture with the surfaces of the teeth further comprises disposing the dental tray about the teeth to contact the whitening agent with the surfaces of the teeth.

14. The method of any one of claims 11 to 13, further comprising contacting the mixture with the surfaces of the teeth for at least 30 seconds, preferably at least 1 minute, more preferably at least 2 minutes.

**Patentansprüche**

1. Aufhellungszusammensetzung, umfassend:

    eine wasserfreie Komponente die umfasst
    ein Basisgel, wobei das Basisgel ein haftungsverstärkendes Mittel, das weißes Petrolatum ist, und ein Chlorisocyanurat umfasst, wobei das Chlorisocyanurat Dichlorisocyanurat enthält, das wasserfreies Natriumdichlorisocyanurat und/oder Natriumdichlorisocyanuratdihydrat ist, wobei das Chlorisocyanurat in der wasserfreien Komponente in einer Menge von 0,01 Gewichts-% bis 3,0 Gewichts-%, bezogen auf das Gesamtgewicht der wasserfreien Komponente, vorhanden ist; und
    eine wässrige Komponente, die Wasser umfasst.

2. Aufhellungszusammensetzung nach Anspruch 1, wobei das Dichlorisocyanurat Natriumdichlorisocyanurat-Dihydrat ist.

3. Aufhellungszusammensetzung nach einem beliebigen der vorhergehenden Ansprüche, wobei das Basisgel eine Polysiloxanflüssigkeit umfasst.

4. Aufhellungszusammensetzung nach einem beliebigen der vorhergehenden Ansprüche, wobei das Basisgel einen druckempfindlichen Klebstoff umfasst, wobei es sich bei dem druckempfindlichen Klebstoff gegebenenfalls um ein Polyorganosiloxan handelt.

5. Aufhellungszusammensetzung nach einem beliebigen der vorhergehenden Ansprüche, wobei das Basisgel ein Verdickungsmittel umfasst, wobei das Verdickungsmittel gegebenenfalls eines oder mehrere aus kolloidalem Silica, pyrogenem Silica, einem vernetzten Polyvinylpyrrolidon (PVP)-Polymer oder Kombinationen davon umfasst.

6. Aufhellungszusammensetzung nach einem beliebigen der vorhergehenden Ansprüche, wobei die wässrige Komponente ein hydrophiles Polymer umfasst, wobei das hydrophile Polymer gegebenenfalls ein Carboxypolymethylen umfasst.

7. Aufhellungszusammensetzung nach einem beliebigen der vorhergehenden Ansprüche, wobei die wässrige Komponente eines oder mehrere von einem Grundiermittel, einem Puffermittel oder Kombinationen davon umfasst, wobei das Grundiermittel gegebenenfalls Natriumhydroxid umfasst, und wobei das Puffermittel weiterhin gegebenenfalls Natriumphosphat monobasisch umfasst.

8. Aufhellungszusammensetzung nach einem beliebigen der vorhergehenden Ansprüche, wobei die Aufhellungszusammensetzung im Wesentlichen frei von Peroxiden und Peroxidverbindungen ist.

9. Aufhellungszusammensetzung nach einem beliebigen der vorhergehenden Ansprüche, wobei das Gewichtsverhältnis der wasserfreien Komponente zur wässrigen Komponente 0,1:1 bis 1,5:1 beträgt.

10. Mundpflegeprodukt, umfassend:

    eine beliebige der Aufhellungszusammensetzungen nach Ansprüchen 1 bis 9; und
    eine Dentalschale, das ein Reservoir definiert, das so ausgelegt ist, dass es eine beliebige der Aufhellungszusammensetzungen nach den Ansprüchen 1 bis 9 enthält.

11. Verfahren zum Aufhellen von Zähnen, umfassend:

    Inkontaktbringen der wasserfreien Komponente und der wässrigen Komponente einer beliebigen der Aufhellungszusammensetzungen nach den Ansprüchen 1 bis 9 miteinander, um eine Mischung zu bilden; und
    Inkontaktbringen der Mischung mit Oberflächen der Zähne.

**12.** Verfahren nach Anspruch 11, wobei das Inkontaktbringen der Mischung mit den Zahnoberflächen das Einbringen des Gemischs in eine Dentalschale umfasst.

**13.** Verfahren nach Anspruch 12, wobei das Inkontaktbringen der Mischung mit den Oberflächen der Zähne weiterhin das Anordnen der Dentalschale um die Zähne herum umfasst, um das Aufhellungsmittel mit den Oberflächen der Zähne in Kontakt zu bringen.

**14.** Verfahren nach einem beliebigen der Ansprüche 11 bis 13, weiterhin umfassend das Inkontaktbringen der Mischung mit den Oberflächen der Zähne für mindestens 30 Sekunden, vorzugsweise mindestens 1 Minute, noch bevorzugter mindestens 2 Minuten.

**Revendications**

**1.** Composition de blanchiment comprenant :

un constituant anhydre comprenant
un gel de base, dans lequel le gel de base comprend un agent améliorant l'adhérence, qui est de la vaseline blanche, et un chloroisocyanurate, dans lequel le chloroisocyanurate comprend du dichloroisocyanurate, qui est le dichloroisocyanurate de sodium anhydre et/ou le dichloroisocyanurate de sodium dihydraté, dans lequel le chloroisocyanurate est présent dans le constituant anhydre en une quantité comprise entre 0,01 % en poids et 3,0 % en poids, par rapport au poids total du constituant anhydre ; et
un constituant aqueux comprenant de l'eau.

**2.** Composition de blanchiment selon la revendication 1, dans laquelle le dichloroisocyanurate est le dichloroisocyanurate de sodium dihydraté.

**3.** Composition de blanchiment selon l'une quelconque des revendications précédentes, dans laquelle le gel de base comprend un fluide à base de polysiloxane.

**4.** Composition de blanchiment selon l'une quelconque des revendications précédentes, dans laquelle le gel de base comprend un adhésif sensible à la pression, éventuellement, l'adhésif sensible à la pression est un polyorganosiloxane.

**5.** Composition de blanchiment selon l'une quelconque des revendications précédentes, dans laquelle le gel de base comprend un épaississant, éventuellement, l'épaississant comprend un ou plusieurs éléments parmi la silice colloïdale, la silice sublimée, un polymère de polyvinylpyrrolidone (PVP) réticulée ou des combinaisons de ceux-ci.

**6.** Composition de blanchiment selon l'une quelconque des revendications précédentes, dans laquelle le constituant aqueux comprend un polymère hydrophile, éventuellement, le polymère hydrophile comprend un carboxypolyméthylène.

**7.** Composition de blanchiment selon l'une quelconque des revendications précédentes, dans laquelle le constituant aqueux comprend un ou plusieurs éléments parmi un agent alcalinisant, un agent tampon ou des combinaisons de ceux-ci, éventuellement, l'agent alcalinisant comprend de l'hydroxyde de sodium, en outre éventuellement, l'agent tampon comprend du phosphate de sodium monobasique.

**8.** Composition de blanchiment selon l'une quelconque des revendications précédentes, dans laquelle la composition de blanchiment est sensiblement exempte de peroxydes et de composés de peroxyde.

**9.** Composition de blanchiment selon l'une quelconque des revendications précédentes, dans laquelle un rapport pondéral entre le constituant anhydre et le constituant aqueux est compris entre 0,1:1 et 1,5:1.

**10.** Produit de soins bucco-dentaires, comprenant :

l'une quelconque des compositions de blanchiment selon les revendications 1 à 9 ; et
une gouttière dentaire définissant un réservoir configuré pour contenir l'une quelconque des compositions de blanchiment selon les revendications 1 à 9.

**11.** Procédé de blanchiment des dents, comprenant :

la mise en contact du constituant anhydre et du constituant aqueux de l'une quelconque des compositions de blanchiment selon les revendications 1 à 9, l'un avec l'autre, pour former un mélange ; et la mise en contact du mélange avec les surfaces des dents.

**12.** Procédé selon la revendication 11, dans lequel la mise en contact du mélange avec les surfaces des dents comprend la mise en place du mélange dans une gouttière dentaire.

**13.** Procédé selon la revendication 12, dans lequel la mise en contact du mélange avec les surfaces des dents comprend en outre la mise en place de la gouttière dentaire autour des dents pour mettre l'agent de blanchiment en contact avec les surfaces des dents.

**14.** Procédé selon l'une quelconque des revendications 11 à 13, comprenant en outre la mise en contact du mélange avec les surfaces des dents pendant au moins 30 secondes, de préférence au moins 1 minute, et plus préférablement au moins 2 minutes.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20100012891 A1

**Non-patent literature cited in the description**

- **THAU et al.** A New Procedure for the Preparation of Polyethylene-Mineral Oil Gels. *J. Soc. Cosmetic Chemists*, 1965, vol. 16, 359-363